# EUROPEAN PATENT APPLICATION

(11) **EP 2 213 682 A1**
(43) Date of publication of application: **04.08.2010**
(21) Application number: 09305092.0
(22) Date of filing: 30.01.2009
(51) Int. Cl.: C07K 14/715, C07K 16/24, A61K 38/17, A61P 37/02, A61P 35/00, A61P 31/12, G01N 33/53, G01N 33/574

(54) **TSLP promotes immune evasion and persistence of viruses**

(71) Applicant: Institut Curie, 75248 Paris Cedex 05 (FR)
(72) Inventor: Soumelis, Vassili, 75013 Paris (FR); Fernandez, Isabel, 75013 Paris (FR)
(74) Representative: Blot, Philippe Robert Emile

(57) **Abstract**

The invention relates to the treatment or prevention of a chronic viral infection with a Thymic Stromal Lymphopoietin (TSLP) antagonist thereby avoiding immune evasion and persistence of the virus. The invention also provides a method of prognosing the evolution of a cervical dysplasia by TSLP expression in a sample of said cervical dysplasia.

SEQUENCE LISTING

<110>  Institut Curie

<120>  TSLP promotes immune evasion and persistence of viruses

<130>  BET08P0912

<160>  5

<170>  PatentIn version 3.4

<210>  1
<211>  743
<212>  DNA
<213>  Homo sapiens


<220>
<221>  misc_feature
<222>  (1)..(743)
<223>  thymic stromal lymphopoietin (TSLP)

<220>
<221>  CDS
<222>  (200)..(679)

<400>  1
gcagccagaa agctctggag catcagggag actccaactt aaggcaacag catgggtgaa      60

taagggcttc ctgtggactg gcaatgagag gcaaaacctg gtgcttgagc actggcccct     120

aaggcaggcc ttacagatct cttacactcg tggtgggaag agtttagtgt gaaactgggg     180

tggaattggg tgtccacgt atg ttc cct ttt gcc tta cta tat gtt ctg tca      232
                     Met Phe Pro Phe Ala Leu Leu Tyr Val Leu Ser
                     1               5                   10

gtt tct ttc agg aaa atc ttc atc tta caa ctt gta ggg ctg gtg tta      280
Val Ser Phe Arg Lys Ile Phe Ile Leu Gln Leu Val Gly Leu Val Leu
            15                  20                  25

act tac gac ttc act aac tgt gac ttt gag aag att aaa gca gcc tat      328
Thr Tyr Asp Phe Thr Asn Cys Asp Phe Glu Lys Ile Lys Ala Ala Tyr
        30                  35                  40

ctc agt act att tct aaa gac ctg att aca tat atg agt ggg acc aaa      376
Leu Ser Thr Ile Ser Lys Asp Leu Ile Thr Tyr Met Ser Gly Thr Lys
    45                  50                  55

agt acc gag ttc aac aac acc gtc tct tgt agc aat cgg cca cat tgc      424
Ser Thr Glu Phe Asn Asn Thr Val Ser Cys Ser Asn Arg Pro His Cys
60                  65                  70                  75

ctt act gaa atc cag agc cta acc ttc aat ccc acc gcc ggc tgc gcg      472
Leu Thr Glu Ile Gln Ser Leu Thr Phe Asn Pro Thr Ala Gly Cys Ala
                80                  85                  90

tcg ctc gcc aaa gaa atg ttc gcc atg aaa act aag gct gcc tta gct      520
Ser Leu Ala Lys Glu Met Phe Ala Met Lys Thr Lys Ala Ala Leu Ala
            95                  100                 105

atc tgg tgc cca ggc tat tcg gaa act cag ata aat gct act cag gca      568
Ile Trp Cys Pro Gly Tyr Ser Glu Thr Gln Ile Asn Ala Thr Gln Ala
        110                 115                 120

atg aag aag agg aga aaa agg aaa gtc aca acc aat aaa tgt ctg gaa      616
Met Lys Lys Arg Arg Lys Arg Lys Val Thr Thr Asn Lys Cys Leu Glu
    125                 130                 135

caa gtg tca caa tta caa gga ttg tgg cgt cgc ttc aat cga cct tta      664
Gln Val Ser Gln Leu Gln Gly Leu Trp Arg Arg Phe Asn Arg Pro Leu
140                 145                 150                 155

ctg aaa caa cag taa accatcttta ttatggtcat atttcacagc ccaaaataaa      719
Leu Lys Gln Gln

tcatctttat taagtaaaaa aaaa                                           743

<210>  2
<211>  159
<212>  PRT
<213>  Homo sapiens

<400>  2

Met Phe Pro Phe Ala Leu Leu Tyr Val Leu Ser Val Ser Phe Arg Lys
1               5                   10                  15

Ile Phe Ile Leu Gln Leu Val Gly Leu Val Leu Thr Tyr Asp Phe Thr
            20                  25                  30

Asn Cys Asp Phe Glu Lys Ile Lys Ala Ala Tyr Leu Ser Thr Ile Ser
        35                  40                  45

Lys Asp Leu Ile Thr Tyr Met Ser Gly Thr Lys Ser Thr Glu Phe Asn
    50                  55                  60

Asn Thr Val Ser Cys Ser Asn Arg Pro His Cys Leu Thr Glu Ile Gln
65                  70                  75                  80

Ser Leu Thr Phe Asn Pro Thr Ala Gly Cys Ala Ser Leu Ala Lys Glu
                85                  90                  95

Met Phe Ala Met Lys Thr Lys Ala Ala Leu Ala Ile Trp Cys Pro Gly
            100                 105                 110

Tyr Ser Glu Thr Gln Ile Asn Ala Thr Gln Ala Met Lys Lys Arg Arg
        115                 120                 125

Lys Arg Lys Val Thr Thr Asn Lys Cys Leu Glu Gln Val Ser Gln Leu
    130                 135                 140

Gln Gly Leu Trp Arg Arg Phe Asn Arg Pro Leu Leu Lys Gln Gln
145                 150                 155

<210>  3
<211>  1116
<212>  DNA
<213>  Homo sapiens

<220>
<221>  misc_feature
<222>  (1)..(1116)
<223>  thymic stromal lymphopoietin protein receptor (TSLPR)

<220>
<221>  CDS
<222>  (1)..(1116)

<400>  3
atg ggg cgg ctg gtt ctg ctg tgg gga gct gcc gtc ttt ctg ctg gga      48
Met Gly Arg Leu Val Leu Leu Trp Gly Ala Ala Val Phe Leu Leu Gly
1               5                   10                  15

ggc tgg atg gct ttg ggg caa gga gga gca gca gaa gga gta cag att      96
Gly Trp Met Ala Leu Gly Gln Gly Gly Ala Ala Glu Gly Val Gln Ile
            20                  25                  30

cag atc atc tac ttc aat tta gaa acc gtg cag gtg aca tgg aat gcc      144
Gln Ile Ile Tyr Phe Asn Leu Glu Thr Val Gln Val Thr Trp Asn Ala
        35                  40                  45

agc aaa tac tcc agg acc aac ctg act ttc cac tac aga ttc aac ggt      192
Ser Lys Tyr Ser Arg Thr Asn Leu Thr Phe His Tyr Arg Phe Asn Gly
    50                  55                  60

gat gag gcc tat gac cag tgc acc aac tac ctt ctc cag gaa ggt cac      240
Asp Glu Ala Tyr Asp Gln Cys Thr Asn Tyr Leu Leu Gln Glu Gly His
65                  70                  75                  80

act tcg ggg tgc ctc cta gac gca gag cag cga gac gac att ctc tat      288
Thr Ser Gly Cys Leu Leu Asp Ala Glu Gln Arg Asp Asp Ile Leu Tyr
                85                  90                  95

ttc tcc atc agg aat ggg acg cac ccc gtt ttc acc gca agt cgc tgg      336
Phe Ser Ile Arg Asn Gly Thr His Pro Val Phe Thr Ala Ser Arg Trp
            100                 105                 110

atg gtt tat tac ctg aaa ccc agt tcc ccg aag cac gtg aga ttt tcg      384
Met Val Tyr Tyr Leu Lys Pro Ser Ser Pro Lys His Val Arg Phe Ser
        115                 120                 125

tgg cat cag gat gca gtg acg gtg acg tgt tct gac ctg tcc tac ggg      432
Trp His Gln Asp Ala Val Thr Val Thr Cys Ser Asp Leu Ser Tyr Gly
    130                 135                 140

gat ctc ctc tat gag gtt cag tac cgg agc ccc ttc gac acc gag tgg      480
Asp Leu Leu Tyr Glu Val Gln Tyr Arg Ser Pro Phe Asp Thr Glu Trp
145                 150                 155                 160

cag tcc aaa cag gaa aat acc tgc aac gtc acc ata gaa ggc ttg gat      528
Gln Ser Lys Gln Glu Asn Thr Cys Asn Val Thr Ile Glu Gly Leu Asp
                165                 170                 175

gcc gag aag tgt tac tct ttc tgg gtc agg gtg aag gct atg gag gat      576
Ala Glu Lys Cys Tyr Ser Phe Trp Val Arg Val Lys Ala Met Glu Asp
            180                 185                 190

gta tat ggg cca gac aca tac cca agc gac tgg tca gag gtg aca tgc      624
Val Tyr Gly Pro Asp Thr Tyr Pro Ser Asp Trp Ser Glu Val Thr Cys
        195                 200                 205

tgg cag aga ggc gag att cgg gac gcc tgt gca gag aca cca acg cct      672
Trp Gln Arg Gly Glu Ile Arg Asp Ala Cys Ala Glu Thr Pro Thr Pro
    210                 215                 220

ccc aaa cca aag ctg tcc aaa ttt att tta att tcc agc ctg gcc atc      720
Pro Lys Pro Lys Leu Ser Lys Phe Ile Leu Ile Ser Ser Leu Ala Ile
225                 230                 235                 240

ctt ctg atg gtg tct ctc ctc ctt ctg tct tta tgg aaa tta tgg aga      768
Leu Leu Met Val Ser Leu Leu Leu Leu Ser Leu Trp Lys Leu Trp Arg
                245                 250                 255

gtg aag aag ttt ctc att ccc agc gtg cca gac ccg aaa tcc atc ttc          816
Val Lys Lys Phe Leu Ile Pro Ser Val Pro Asp Pro Lys Ser Ile Phe
            260                 265                 270

ccc ggg ctc ttt gag ata cac caa ggg aac ttc cag gag tgg atc aca          864
Pro Gly Leu Phe Glu Ile His Gln Gly Asn Phe Gln Glu Trp Ile Thr
        275                 280                 285

gac acc cag aac gtg gcc cac ctc cac aag atg gca ggt gca gag caa          912
Asp Thr Gln Asn Val Ala His Leu His Lys Met Ala Gly Ala Glu Gln
    290                 295                 300

gaa agt ggc ccc gag gag ccc ctg gta gtc cag ttg gcc aag act gaa          960
Glu Ser Gly Pro Glu Glu Pro Leu Val Val Gln Leu Ala Lys Thr Glu
305                 310                 315                 320

gcc gag tct ccc agg atg ctg gac cca cag acc gag gag aaa gag gcc         1008
Ala Glu Ser Pro Arg Met Leu Asp Pro Gln Thr Glu Glu Lys Glu Ala
                325                 330                 335

tct ggg gga tcc ctc cag ctt ccc cac cag ccc ctc caa ggc ggt gat         1056
Ser Gly Gly Ser Leu Gln Leu Pro His Gln Pro Leu Gln Gly Gly Asp
            340                 345                 350

gtg gtc aca atc ggg ggc ttc acc ttt gtg atg aat gac cgc tcc tac         1104
Val Val Thr Ile Gly Gly Phe Thr Phe Val Met Asn Asp Arg Ser Tyr
        355                 360                 365

gtg gcg ttg tga                                                         1116
Val Ala Leu
    370


<210>  4
<211>  371
<212>  PRT
<213>  Homo sapiens

<400>  4

Met Gly Arg Leu Val Leu Leu Trp Gly Ala Ala Val Phe Leu Leu Gly
1               5                   10                  15

Gly Trp Met Ala Leu Gly Gln Gly Gly Ala Ala Glu Gly Val Gln Ile
            20                  25                  30

Gln Ile Ile Tyr Phe Asn Leu Glu Thr Val Gln Val Thr Trp Asn Ala
        35                  40                  45

Ser Lys Tyr Ser Arg Thr Asn Leu Thr Phe His Tyr Arg Phe Asn Gly
    50                  55                  60

Asp Glu Ala Tyr Asp Gln Cys Thr Asn Tyr Leu Leu Gln Glu Gly His
65                  70                  75                  80

Thr Ser Gly Cys Leu Leu Asp Ala Glu Gln Arg Asp Asp Ile Leu Tyr
                85                  90                  95

Phe Ser Ile Arg Asn Gly Thr His Pro Val Phe Thr Ala Ser Arg Trp
            100                 105                 110

Met Val Tyr Tyr Leu Lys Pro Ser Ser Pro Lys His Val Arg Phe Ser
        115                 120                 125

Trp His Gln Asp Ala Val Thr Val Thr Cys Ser Asp Leu Ser Tyr Gly
    130                 135                 140

Asp Leu Leu Tyr Glu Val Gln Tyr Arg Ser Pro Phe Asp Thr Glu Trp
145                 150                 155                 160

Gln Ser Lys Gln Glu Asn Thr Cys Asn Val Thr Ile Glu Gly Leu Asp
                165                 170                 175

Ala Glu Lys Cys Tyr Ser Phe Trp Val Arg Val Lys Ala Met Glu Asp
            180                 185                 190

Val Tyr Gly Pro Asp Thr Tyr Pro Ser Asp Trp Ser Glu Val Thr Cys
        195                 200                 205

Trp Gln Arg Gly Glu Ile Arg Asp Ala Cys Ala Glu Thr Pro Thr Pro
    210                 215                 220

Pro Lys Pro Lys Leu Ser Lys Phe Ile Leu Ile Ser Ser Leu Ala Ile
225                 230                 235                 240

Leu Leu Met Val Ser Leu Leu Leu Leu Ser Leu Trp Lys Leu Trp Arg
                245                 250                 255

Val Lys Lys Phe Leu Ile Pro Ser Val Pro Asp Pro Lys Ser Ile Phe
            260                 265                 270

Pro Gly Leu Phe Glu Ile His Gln Gly Asn Phe Gln Glu Trp Ile Thr
        275                 280                 285

Asp Thr Gln Asn Val Ala His Leu His Lys Met Ala Gly Ala Glu Gln
    290                 295                 300

Glu Ser Gly Pro Glu Glu Pro Leu Val Val Gln Leu Ala Lys Thr Glu
305                 310                 315                 320

Ala Glu Ser Pro Arg Met Leu Asp Pro Gln Thr Glu Glu Lys Glu Ala
                325                 330                 335

Ser Gly Gly Ser Leu Gln Leu Pro His Gln Pro Leu Gln Gly Gly Asp
            340                 345                 350

Val Val Thr Ile Gly Gly Phe Thr Phe Val Met Asn Asp Arg Ser Tyr
        355                 360                 365

Val Ala Leu
    370

<210>  5

<211>  459
<212>  PRT
<213>  Homo sapiens


<220>
<221>  SIGNAL
<222>  (1)..(20)

<220>
<221>  MISC_FEATURE
<222>  (21)..(459)
<223>  Interleukin-7 receptor subunit alpha

<220>
<221>  MISC_FEATURE
<222>  (21)..(239)
<223>  extracellular domain

<400>  5

## Description

The invention relates to the treatment or prevention of a chronic viral infection with a Thymic Stromal Lymphopoietin (TSLP) antagonist thereby avoiding immune evasion and persistence of the virus. The invention also provides a method of prognosing the evolution of a cervical dysplasia by detecting TSLP expression in a sample of said cervical dysplasia.

The integrity of the epithelium that covers body surfaces at the interface with the external environment is essential for an optimal host protection against noxious factors and pathogens.

Human papilloma virus (HPV) is a non-lytic, double-stranded DNA, virus that infects the basal cell layer of the stratified squamous epithelia of skin and mucosa.

Most HPV lesions spontaneously regress without further consequences for the host. However, HPV can cause persistent disease for months or years in immunocompetent hosts, despite producing immunogenic proteins throughout its replicative cycle. In some cases, the infection gives rise to a permanent lesion, which can ultimately progress to cervical intraepithelial neoplasia and cervical cancer. Anogenital and cutaneous squamous cell carcinoma have also been linked to HPV infection.

More specifically, non-oncogenic (low-risk) HPV subtypes may give rise to benign lesions, such as warts (verruca) or condyloma, whereas oncogenic (high-risk) subtypes, especially HPV type 16 (HPV-16) and HPV type 18 (HPV-18), are the causal agents of uterine cervical dysplasia and cancer (Kanodia et al., Curr Cancer Drug Targets, 2007; 7, 79-89, Stanley, Vaccine, 2006; 24, S16-S22).

Consequently, the chronic nature of the infection, in association with high-risk oncogenic types of HPV, results in an increased risk of cellular transformation and malignancy.

Therefore, HPV infection has become a major public health issue worldwide and it is crucial to better understand its physiopathology in order to optimise the diagnosis, follow-up, treatment and prevention of HPV-related pathologies.

The immune response to HPV infection remains incompletely understood. Persistance of the virus has been attributed to various immune escape mechanisms. In genital lesions, HPV infection and replication is restricted to epithelial cells, thus limiting viremia and the contact between the virus and innate immune cells present in the dermis, such as dendritic cells (DC). HPV is not cytolytic, and does not induce the release of "danger" signals and pro-inflammatory cytokines by epithelial cells. Moreover, high-risk HPV have evolved mechanisms to inhibit type I IFN production by infected cells and type I IFN-inducible gene expression. As a result, it has been suggested that the host can remain ignorant of the pathogen (Stanley, Vaccine, 2006; 24, S16-S22).

However, several lines of evidence indicate that an immune response takes place in HPV infections:
1) they are self-limited and most of them spontaneously regress;
2) their incidence and progression is increased in immunosuppressed patients;
3) signs of CD4 and CD8 T cell responses have been observed in regressing lesions *in situ* (Coleman, Am. J. Clin. Pathol. 1994;102(6):768-74) and systemically (van Poelgeest MI, Int. J. Cancer. 2006;118(3):675-83).

Yet current mechanisms of viral immune escape do not explain how such immune responses can be initiated.

Since HPV is only permissive for viral replication in epidermal keratinocytes, the ability of the virus to influence the immune system must be limited to the localized environment of the infected epidermis.

Langerhans cells (LC) are the resident epidermal DC and constitute the primary antigen-presenting cells (APC) in the skin. Immature LC form a contiguous network throughout the epithelium. Langerhans cells are capable of capturing antigens and migrate to the skin-draining lymph nodes in response to several stimuli. Thus, LC are essential for the initiation of an adaptive immune response against viral antigens encountered within the epidermis and an increase in susceptibility to disease has been demonstrated when these cells are reduced in number or absent from the skin. In particular, LC have been shown to play an important role in the defence against viral infections, such as HIV and herpes simplex virus (HSV).

Under steady-state conditions LC numbers are homeostatically maintained, but homeostasis is disrupted after epidermal viral infections. In the context of vaccinia virus infection of murine skin, a net increase in LC had been observed in infected epidermis which would reflect the disruption of homeostasis as a result of increased immigration of LC into the skin in response to proinflammatory cytokines, counterbalancing the increase in emigration of antigen loaded LC from the skin.

In contrast, in cervical HPV-related lesions, a net decrease in LC in HPV-infected epidermis is observed which results directly from the virus infection (Tay et al., Br. J. Obstet. Gynaecol. 1987, 94(11):1094-7; Matthews et al., J. Virol., 2003, 77(15):8378-8385). Depletion of LC from the epithelium has been considered as a relevant strategy to avoid the host immune response (Stanley, Vaccine, 2006, 24, S16-S22). However, the factors triggering the emigration of LCs have remained unknown.

In an attempt to dissect the innate immune response to HPV, the Inventors decided to focus on the factors that activate DC, as this step is critical for the induction and shaping of an adaptive immune response.

The inventors found that epithelial cells expressed the pro-allergic cytokine TSLP in HPV infected lesions and that TSLP production correlated with LC depletion.

Human Thymic Stromal Lymphopoietin Protein (TSLP) is a cytokine of the IL-7 family produced by epithelial cells. TSLP production is upregulated in atopic dermatitis and has been involved in the activation of DC to induce a pro-inflammatory Th2 response *in vitro* and *in vivo.* A role for TSLP in DC migration has been suggested in the context of atopic dermatitis (Soumelis et al., Nature Immunology, 2002, 3, 673-680; Soumelis, Médecine/Sciences, 2007, 23(8-9), 692-694; Ebner et al., J. Allergy Clin. Immunol., 119,(4), 982-990), although a direct role of TSLP was not formally demonstrated. Indeed, TSLP was reported to increase migration of migratory LCs from epidermal explant cultures (Ebner et al., J. Allergy Clin. Immunol., 119,(4), 982-990) but this model does not make it possible to preclude the involvement of other factors produced by the keratinocytes present in the epidermal sheets. Furthermore, LCs are not depleted in atopic dermatitis lesions despite the presence of TSLP in these lesions.

Strikingly, the Inventors have now demonstrated that TSLP directly triggers DC migration *ex vivo,* independently of any other chemokine, by the ability to polarize both the microtubule and actinomyosin cytoskeleton of DC. This result indicates a critical function for TSLP in HPV infection and suggests that TSLP can be targeted to redirect the local immune response.

Overall, the Inventors demonstrated that the pro-allergic cytokine TSLP promotes immune evasion during HPV infection through: (1) immune deviation towards the Th2 phenotype and (2) depletion from epithelia of Langerhans cells (LC).

It was previously suggested that the immune system can remain ignorant of the HPV virus because of the absence of local danger signals (Stanley, Vaccine, 2006, 24, S16-S22). However, this model fails to explain how most HPV infections are self-limited and spontaneously regress, even after months or years (Stanley, Vaccine, 2006, 24, S16-S22), and how HPV-specific immune responses can be initiated. The TSLP-driven model described herein reconciles these views. Initially, TSLP would activate resident LCs and enable the initiation of anti-HPV immunity (Offringa et al., Curr Top Microbiol Immunol, 2003, 276, 215-240). On a longer term, TSLP would subvert the immune response by promoting chronic LC depletion and immune deviation towards a Th2 response, which is not appropriate for efficient viral clearance. The balance between immunity and immune subversion mechanisms would ultimately determine the outcome of the lesion, as is observed for some common warts, or persistence for many months or years, as is the case for uterine cervical lesions.

TSLP was additionally found to be expressed in skin lesions associated with infection by another virus, the poxvirus Molluscum contagiosum (MCV). In patients without severe immune suppression, lesions produced by MCV typically regress spontaneously, usually within six months to five years. However, Molluscum contagiosum may be more persistent in immunosuppressed patients such as HIV patients.

Accordingly, these results demonstrate for the first time that TSLP makes part of the host's response to viral infection and contributes to an inappropriate immune response, thereby leading to immune evasion and persistence of the virus.

It is thus proposed to block TSLP activity in order to prevent or treat a chronic active viral infection.

### Definitions

"TSLP" denotes "Thymic Stromal Lymphopoietin Protein". TSLP was originally identified in the conditioned medium of a thymic stromal cell line that supported the development of murine IgM⁺ B-cells from fetal liver hematopoietic progenitor cells (Friend et al., Exp. Hematol., 1994, 22:321-328). Cloning of mouse TSLP from a thymic stromal cell line was described by Sims et al. (J. Exp. Med. 2000, 192(5), 671-680). Cloning and sequencing of human TSLP were described in Quentmeier et al. (Leukemia, 2001, 15:1286-1292). The polynucleotide and amino acid sequences of human TSLP are shown in SEQ ID NO: 1 and 2, respectively.

TSLP was found to bind with low affinity to a receptor chain from the hematopoietin receptor family ("TSLP receptor" or "TSLPR"). The murine and human TSLP receptors have been described in U.S. patent application publication No: 2002/0068323. The polynucleotide and amino acid sequences of TSLPR are shown in SEQ ID NO: 3 and 4, respectively. The soluble domain of the TSLPR is approximately amino acids 25 through 231 of SEQ ID NO: 4.

Additionally, TSLP binds with high affinity to a heterodimeric receptor complex composed of the thymic stromal lymphopoietin receptor and the IL-7R alpha chain (Park et al., J. Exp. Med., 2000, 192(5):659-70) ("TSLPR complex"). The amino acid sequence of the human IL-7 receptor alpha chain is shown in SEQ ID NO: 5. The sequence of the soluble domain of the IL-7 receptor alpha consists of amino acids 21 to 239 of SEQ ID NO: 5.

Upon TSLP binding, TSLPR transmits signals towards STAT activation. In particular, TSLP has been shown to induce activation and phosphorylation of STAT-3 and STAT-5 without an involvement of Janus kinases (Sebastian et al. Cell Commun Signal. 2008; 6: 5).

As used herein, the term "subject" or "host" denotes a human or non-human mammal, such as a rodent, a feline, a canine, or a primate.

In the context of the invention, the term "treating" or "treatment", as used herein, is used herein to characterize a method or process that is aimed at (1) delaying or preventing the onset of a disorder or condition to which such term applies; (2) slowing down or stopping the progression, aggravation, or deterioration of the symptoms of the disease state or condition to which such term applies; (3) alleviating or bringing about ameliorations of the symptoms of the disease state or condition to which such term applies; and/or (4) reversing or curing the disease state or condition to which such term applies. A treatment may be administered prior to the onset of the disease, for a prophylactic or preventive action. Alternatively or additionally, a treatment may be administered after initiation of the disease or condition, for a therapeutic action.

### Treatment of a chronic viral infection by blocking TSLP activity

It was demonstrated for the first time by the inventors that TSLP promotes immune evasion following viral infection by deviating the immune response towards a Th2 phenotype, which is an inappropriate response to a viral infection, thereby enabling the active virus to persist in the infected host.

A virus may persist in an organism because the immune response is not sufficient to completely eliminate infected cells and block viral replication. There are two modes of viral persistence: latent infections and chronic infections.

Latent infections are observed with viruses that are capable to integrate their viral genome in the cellular genome. This is the case for instance of *Herpesviridae* (HSV, CMV, EBV, VZV). Several mechanisms may lead to viral genome reactivation, thereby inducing a new viral replication in the host and causing recurrent infections.

In the case of "chronic infections", the virus persists and keeps on replicating despite the immune response. Hence, in chronic infections the virus remains active. As used herein, and by contrast with acute viral infections, an infection is "chronic" when the viral infection persists over at least one month. Examples of viruses likely to cause chronic infection include human papilloma Viruses (HPV), hepatitis viruses (in particular HBV, HCV), human immunodeficiency viruses (HIV), molluscum contagiosum virus (MCV).

Accordingly, the invention provides a method of treating or preventing a chronic viral infection which comprises administering a TSLP antagonist, or a composition thereof, to a subject in need thereof.

The invention also relates to the use of a TSLP antagonist, or a composition thereof, for the manufacture of a medicament intended for treating or preventing a chronic viral infection.

Additionally, the invention concerns a TSLP antagonist, or a composition thereof, for treating or preventing a chronic viral infection.

Said chronic viral infection may be selected from the group consisting of an infection with human papilloma virus (HPV), hepatitis viruses (HBV, HCV), human immunodeficiency viruses (HIV), and molluscum contagiosum virus (MCV).

The Inventors also found that, during HPV infection, TSLP is involved in the depletion from epithelia of Langerhans cells (LC), which is considered as a relevant strategy for the virus to escape the host immune response. Therefore, in the context of HPV infection TSLP may lead to immune escape and virus persistence by two different mechanisms of action.

Thus, according to a preferred embodiment, the virus is a human papilloma virus. Preferably, a HPV infection which may be treated or prevented according to the invention is an infection with a high-risk subtype of HPV, in particular type-16 HPV or type-18 HPV. A "high-risk subtype of HPV" denotes HPV strains which can be the causal agents of uterine cervical dysplasia and cancer.

As used herein the term TSLP "antagonist" or "antagonistic agent" according to the present invention refers to an agent (i.e., molecule) which inhibits or blocks the activity of TSLP. The term "antagonist" is used synonymously with the term "inhibitory agent". The antagonists of the present invention act by blocking or reducing TSLP functional activity. This may be achieved by interfering with TSLP binding to its receptors, or by reducing or preventing expression of TSLP or its receptors, both of which ultimately result in blocking or reducing TSLP signal transduction, hence in blocking or reducing TSLP functional activity.

As referred herein, "TSLP functional activity" may denote, among others, (i) activation of CD11c⁺ dendritic cells, as may be determined by detecting upregulation of activation markers HLA-DR, CD40, CD80, CD86 and CD83, (ii) B cell growth factor activity, and (iii) induction of secretion of Th2-type cytokines (IL-4, IL-5, IL-6, IL-10 and IL-13).

The TSLP antagonists according to the invention are capable of inhibiting or eliminating the functional activity of TSLP *in vivo* and/or *in vitro.* The antagonist may inhibit the functional activity of TSLP by at least about 10%, preferably by at least about 30%, preferably by at least about 50%, preferably by at least about 70, 75 or 80%, still preferably by 85, 90, 95, or 100%.

Functional activity of TSLP may be readily assessed by the one skilled in the art according to known methods. For instance, TSLP activities can be measured in an assay using BAF cells expressing human TSLPR (BAF/HTR), which require active TSLP for proliferation as described in the PCT patent application WO 03/032898. The BAF/HTR bioassay utilizes a murine pro B lymphocyte cell line, which has been transfected with the human TSLP receptor (cell line obtained from Steven F. Ziegler, Benaroya Research Center, Seattle, Wash). The BAF/HTR cells are dependent upon human TSLP (huTSLP) for growth, and proliferate in response to active huTSLP added in test samples. Following an incubation period, cell proliferation is measured by the addition of Alamar Blue dye I. Metabolically active BAF/HRT cells take up and reduce Alamar Blue, which leads to change in the fluorescent properties of the dye. Additional assays for hTSLP activity include, for example, an assay measuring induction of T cell growth from human bone marrow by TSLP as described in U.S. Pat. No. 6,555,520. Another TSLP activity is the ability to activate STAT5 as described in the reference to Levin et al., J. Immunol. 162:677-683 (1999) and PCT application publication WO 03/032898.

Blockade or reduction of TSLP signal transduction may be assayed through the measure of STAT phosphorylation, in particular STAT-3 or STAT-5 phosphorylation. STATs, which are present in the cytoplasm of cells under basal conditions, are activated by phosphorylation on a single tyrosine residue located towards the carboxy terminus of the protein (phosphorylation on Tyr705 in the case of STAT3). Accordingly an inhibitory agent may be identified as an agent which reduces the level of STAT phosphorylation upon TSLP stimulation of a cell expressing TSLPR or the complex receptor TSLPR/ IL-7R alpha chain (the TSLPR complex), as compared with the level of STAT phosphorylation measured in the cell when stimulated with TSLP in the absence of the inhibitory agent. STAT phosphorylation in cells can be readily detected by immunocytochemistry, immunohistochemistry and/or flow cytometry using antibodies which specifically recognize this modification. For instance phosphorylation of STAT3 on tyrosine705 can be detected by immunocytochemistry, immunohistochemistry and/or flow cytometry using commercially available monoclonal or polyclonal antibodies directed against phosphorylated Tyr705-Stat3.

The TSLP antagonists according to the present invention include those which selectively bind to either TSLP or to one or more subunits of a TSLP receptor (i.e. TSLPR, the complex receptor TSLPR/ IL-7R alpha chain, or the TSLPR or IL-7R alpha subunit of the complex receptor TSLPR/ IL-7R alpha chain), thereby reducing or blocking TSLP signal transduction. TSLP antagonists of this type include antibodies or aptamers which bind to TSLP, antibodies or aptamers which bind to one or more subunits of TSLP receptors, peptides (such as peptides of less than about 20 amino acids in length) or polypeptides such as soluble receptors which bind to the cytokine (i.e. soluble TSLP receptor or soluble IL-7R alpha chain) or soluble ligands which bind to the receptor, fusion polypeptides, small molecules, chemicals and peptidomimetics.

As used herein the term "polypeptide" or "peptide" refers to any chain of amino acids linked by peptide bonds, regardless of length or post-translational modification.. Polypeptides include natural proteins, synthetic or recombinant polypeptides and peptides as well as hybrid, post-translationally modified polypeptides, and peptidomimetic. As used herein, the term "amino acid" refers to the 20 standard alpha-amino acids as well as naturally occurring and synthetic derivatives. A polypeptide may contain L or D amino acids or a combination thereof. As used herein the term "peptidomimetic" refers to peptide-like structures which have non-amino acid structures substituted but which mimic the chemical structure of a peptide and retain the functional properties of the peptide. Peptidomimetics may be designed in order to increase peptide stability, bioavailability, solubility, etc.

According to a preferred embodiment, the TSLP antagonist is an antibody which specifically recognizes and binds to TSLP or a fragment thereof, or to a TSLP receptor of a fragment thereof.

As used herein, the terms "antibody" and "immunoglobulin" have the same meaning and are used indifferently in the present invention. Antibody refers to immunoglobulin molecules and immunologically active portions of immunoglobulin molecules, i.e., molecules that contain an antigen binding site that immunospecifically binds an antigen. As such, the term antibody encompasses not only whole antibody molecules, but also antibody fragments as well as variants (including derivatives) of antibodies and antibody fragments.

In natural antibodies, two heavy chains are linked to each other by disulfide bonds and each heavy chain is linked to a light chain by a disulfide bond. Each chain contains distinct sequence domains. The light chain includes two domains, a variable domain (V_{L}) and a constant domain (C_{L}). The heavy chain includes four domains, a variable domain (V_{H}) and three constant domains (C_{H}1, C_{H}2 and C_{H}3, collectively referred to as C_{H}). The variable regions of both light (V_{L}) and heavy (V_{H}) chains determine binding recognition and specificity to the antigen.

The specificity of the antibody resides in the structural complementarity between the antibody combining site and the antigenic determinant. Antibody combining sites are made up of residues that are primarily from the hypervariable or complementarity determining regions (CDRs). They refer to amino acid sequences which, together, define the binding affinity and specificity of the natural Fv region of a native immunoglobulin binding site. The light and heavy chains of an immunoglobulin each have three CDRs, designated L-CDR1, L-CDR2, L-CDR3 and H-CDR1, H-CDR2, H-CDR3, respectively. Therefore, an antigen-binding site includes six CDRs, comprising the CDR set from each of a heavy and a light chain V region.

Framework regions (FRs) refer to amino acid sequences interposed between CDRs, i.e. to those portions of immunoglobulin light and heavy chain variable regions that are relatively conserved among different immunoglobulins in a single species, as defined by Kabat *et al.,* 1991 (Kabat et al., 1991, Sequences of Proteins Of Immunological Interest, National Institute of Health, Bethesda, Md). As used herein, a "human framework region" is a framework region that is substantially identical (about 85%, or more, in particular, 90%, 95% or 100%) to the framework region of naturally occurring human antibody.

The term "monoclonal antibody" or "mAb" as used herein refers to an antibody molecule of a single amino acid composition, that is directed against a specific antigen and which may be produced by a single clone of B cells or hybridoma, or by recombinant methods.

A "humanized antibody" is a chimeric, genetically engineered, antibody in which the CDRs from a mouse antibody ("donor antibody") are grafted onto a human antibody ("acceptor antibody"). Thus, a humanized antibody is an antibody having CDRs from a donor antibody and variable region framework and constant regions from a human antibody. The use of antibody components derived from humanized monoclonal antibodies obviates potential problems associated with the immunogenicity of murine constant regions.

"Antibody fragments" comprise a portion of an intact antibody, preferably the antigen binding or variable region of the intact antibody. Examples of antibody fragments include Fv, Fab, F(ab')₂, Fab', Fd, dAb, dsFv, scFv, sc(Fv)₂, CDRs, diabodies and multi-specific antibodies formed from antibodies fragments.

The term "Fab" denotes an antibody monovalent fragment having a molecular weight of about 50,000 and antigen binding activity, and consisting of the V_{L}, V_{H}, C_{L} and C_{H}1 domains.

The Fv fragment is the N-terminal part of the Fab fragment and consists of the variable portions of one light chain and one heavy chain.

The term "F(ab')₂" refers to an antibody bivalent fragment having a molecular weight of about 100,000 and antigen binding activity, which comprises two Fab fragments linked by a disulfide bridge at the hinge region.

The term "Fab"' refers to an antibody fragment having a molecular weight of about 50,000 and antigen binding activity, which is obtained by cutting a disulfide bond of the hinge region of the F(ab')₂ fragment.

The term "Fd" refers to an antibody fragment consisting of the V_{H} and C_{H}1 domains.

The term "dAb" (Ward et al., 1989 Nature 341:544-546) refers to a single variable domain antibody, i.e. an antibody fragment which consists of a V_{H} or V_{L} domain.

A single chain Fv ("scFv") polypeptide is a covalently linked V_{H}::V_{L} heterodimer which is usually expressed from a gene fusion including V_{H} and V_{L} encoding genes linked by a peptide-encoding linker. "dsFv" is a V_{H}::V_{L} heterodimer stabilised by a disulfide bond. Divalent and multivalent antibody fragments can form either spontaneously by association of monovalent scFvs, or can be generated by coupling monovalent scFvs by a peptide linker, such as divalent sc(Fv)₂.

The term "diabodies" refers to small antibody fragments with two antigen-binding sites, which fragments comprise a V_{H} domain connected to a V_{L} domain in the same polypeptide chain (V_{H}-V_{L}). By using a linker that is too short to allow pairing between the two domains on the same chain, the domains are forced to pair with the complementarity domains of another chain and create two antigen-binding sites.

Antibodies according to the invention may be produced by any technique known in the art, such as, without limitation, any chemical, biological, genetic or enzymatic technique, either alone or in combination. The antibodies of this invention can be obtained by producing and culturing hybridomas.

The skilled person may also use antibodies against TSLP or a TSLP receptor which are commercially available. These include, for instance, an anti-human IL-7Ra antibody, anti-human TSLP and anti-human TSLP-R antibodies. For instance, anti-human IL-7Rα monoclonal (MAB306) and polyclonal (AF-306-PB) antibodies, anti-human TSLP monoclonal (MAB1398) and polyclonal (AF1398) antibodies, anti-human TSLP-R monoclonal (MAB981) and polyclonal (AF981) antibodies are available at R&D Systems. Anti-TSLP-R (M505; Amgen); or anti-TSLP (M385; Amgen) have also been mentioned in the art.

Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library, as described in Tuerk C. and Gold L., Science, 1990, 249(4968):505-10. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., Clin. Chem., 1999, 45(9):1628-50. Peptide aptamers consists of a conformationally constrained antibody variable region displayed by a platform protein, such as E. coli Thioredoxin A that are selected from combinatorial libraries by two hybrid methods (Colas et al., Nature, 1996,380, 548-50).

TSLP antagonists according to the present invention also include molecules which reduce or prevent expression of TSLP or its receptors (TSLPR or the complex receptor TSLPR/ IL-7R alpha chain), such as, for example, antisense oligonucleotides comprising a single-stranded polynucleotide sequence (either RNA or DNA) capable of binding to target mRNA (sense) or DNA (antisense) sequences, and interfering messenger RNA, or ribozymes.

Antisense or sense oligonucleotides comprise fragments of the targeted polynucleotide sequence encoding TSLP or its receptor. Such a fragment generally comprises at least about 14 nucleotides, typically from about 14 to about 30 nucleotides. The ability to derive an antisense or a sense oligonucleotide, based upon a nucleic acid sequence encoding a given protein is described in, for example, Stein and Cohen (Cancer Res., 1988, 48:2659), and van der Krol et al. (BioTechniques, 1988, 6:958).

Binding of antisense or sense oligonucleotides to target nucleic acid sequences results in the formation of duplexes that block or inhibit protein expression by one of several means, including enhanced degradation of the mRNA by RNAse H, inhibition of splicing, premature termination of transcription or translation, or by other means. The antisense oligonucleotides thus may be used to block expression of proteins. Antisense or sense oligonucleotides further comprise oligonucleotides having modified sugar-phosphodiester backbones (or other sugar linkages, such as those described in WO 91/06629) and wherein such sugar linkages are resistant to endogenous nucleases. Such oligonucleotides with resistant sugar linkages are stable *in vivo* (i.e., capable of resisting enzymatic degradation) but retain sequence specificity to be able to bind to target nucleotide sequences.

Other examples of sense or antisense oligonucleotides include those oligonucleotides which are covalently linked to moieties that increases affinity of the oligonucleotide for a target nucleic acid sequence, such as poly-(L)-lysine. Further still, intercalating agents, such as ellipticine, and alkylating agents or metal complexes may be attached to sense or antisense oligonucleotides to modify binding specificities of the antisense or sense oligonucleotide for the target nucleotide sequence.

Antisense or sense oligonucleotides may be introduced into a cell containing the target nucleic acid by any gene transfer method, including, for example, electroporation, microinjection, transduction, cell fusion, DEAE dextran, calcium phosphate precipitation, use of a gene gun, or lipofection, or by using gene transfer vectors such as Epstein-Barr virus or adenovirus.

Sense or antisense oligonucleotides also may be introduced into a cell containing the target nucleic acid by formation of a conjugate with a ligand-binding molecule. Suitable ligand binding molecules include, but are not limited to, cell surface receptors, growth factors, other cytokines, or other ligands that bind to cell surface receptors. Preferably, conjugation of the ligand-binding molecule does not substantially interfere with the ability of the ligand-binding molecule to bind to its corresponding molecule or receptor, or block entry of the sense or antisense oligonucleotide or its conjugated version into the cell.

Additional methods for preventing expression of TSLP or TSLP receptors is RNA interference (RNAi) produced by the introduction of specific small interfering RNA (siRNA), as described, for example in Bosher et al., Nature Cell Biol 2, E31-E36 (2000).

Ribozymes can also function as inhibitors of TSLP or TSLP receptor expression for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of TSLP or TSLP receptor mRNA sequences are thereby useful within the scope of the present invention. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, e.g., ribonuclease protection assays.

Advantageously, an immunostimulating agent may be administered simultaneously or sequentially with the TSLP antagonist to redirect the host immune response towards a Th1-type response and eliminate the virus.

Accordingly, the invention also provides a combination of at least one TSLP antagonist and at least one immunostimulating agent, or a composition thereof, as a medicament. In said combination, said at least one TSLP antagonist and said at least one immunostimulating agent are intended to be administered simultaneously or sequentially.

Accordingly, the invention provides for a method of treating or preventing a chronic viral infection which comprises administering at least one TSLP antagonist and at least one immunostimulating agent, or a composition thereof, to a subject in need thereof, wherein said at least one TSLP antagonist and said at least one immunostimulating agent are administered simultaneously or sequentially.

The invention also relates to the use of a combination of at least one TSLP antagonist and at least one immunostimulatory agent, or a composition thereof, for the manufacture of a medicament, particularly for a medicament intended for treating or preventing a chronic viral infection. In said use, said at least one TSLP antagonist is intended to be administered simultaneously or sequentially with said at least one immunostimulating agent. Accordingly, if the TSLP antagonist and immunostimulating agent are to be administered simultaneously, said medicament may comprise the immunostimulating agent.

The term "combination", as used herein, may be in the form of a kit comprising at least one TSLP antagonist and at least one immunostimulating agent; the components of the kit may be administered simultaneously or sequentially. The term "combination" may also designate the TSLP antagonist and immunostimulating agent as separate products which may be administered simultaneously or sequentially. Alternatively, if the TSLP antagonist and immunostimulating agent are to be administered simultaneously, a composition comprising a TSLP antagonist and immunostimulating agent may be provided.

Said combination, kit, composition of TSLP antagonist and immunostimulating agent as separate products, are intended for the treatment of a chronic viral infection.

The invention further concerns provides a combination of a TSLP antagonist and an immunostimulating agent, wherein the TSLP antagonist and immunostimulating agent are intended to be administered simultaneously or sequentially, for treating or preventing a chronic viral infection. The combination may be in the form of a kit, composition, and TSLP antagonist and immunostimulating agent as separate products, as described above.

Said immunostimulating agent may be a Th-1 cytokine, such as interferon (IFN; in particular IFN-gamma), tumor necrosis factor (TNF; in particular TNF-alpha or TNF-beta), and interleukin-2 (IL-2), or an inducer of the production of a Th-1 cytokine.

Preferably said immunostimulating agent is selected from the group consisting of IFN, IFN-gamma, inducers of IFN, TNF, inducers of TNF, IL-2, and ligands of Toll-like receptors (TLR).

TLRs are a type of pattern recognition receptor (PRR) and recognize molecules that are broadly shared by pathogens but distinguishable from host molecules. Ligands of Toll-like receptors have adjuvant effects on the immune response. For instance TLR3 is activated by ligands of viral origin, in particular by abnormally large amounts of double-stranded RNA (dsRNA) which is normally present in very low quantities in cells, and induces production of IFN. Ligands of TLR3 include for instance polyinosinic-polycytidylic acid (Poly IC, [(2R,3S,4R,5R)-5-(4-amino-2-oxopyrimidin-1-yl)-3,4-dihydroxyoxolan-2-yl]methyl dihydrogen phosphate; [(2R,3S,4R,5R)-3,4-dihydroxy-5-(6-oxo-3H-purin-9-yl)oxolan-2-yl]methyl dihydrogen phosphate) which is a high molecular weight synthetic double stranded RNA, and polyadenylic-polyuridylic acid (Poly AU, [(2R,3S,4R,5R)-5-(6-aminopurin-9-yl)-3,4-dihydroxyoxolan-2-yl]methyl dihydrogen phosphate; [(2R,3S,4R,5R)-5-(2,4-dioxopyrimidin-1-yl)-3,4-dihydroxyoxolan-2-yl]methyl dihydrogen phosphate) which is a double stranded complex of synthetic polyribonucleotides. These TLR3 ligands are IFN inducers.

IFN can also be induced by TLR7, for instance by activation of TLR7 with a ligand such as imidazoquinoline, loxoribine and bropirimine.

Additionally, CpG-ODNs, i.e. oligodeoxynucleotides (ODN) containing unmethylated CpG motifs (cytosine followed by guanosine), induce IFN (type I IFN) and TNF through TLR-9,

Another object of the invention relates to a composition comprising at least one TSLP antagonist, and eventually at least one immunostimulating agent. Accordingly, another object of the invention comprises a pharmaceutical composition, comprising at least one TSLP antagonist, and eventually at least one immunostimulating agent, in combination with a pharmaceutically acceptable excipient. Optionally said pharmaceutical composition may further comprise sustained-release matrices, such as biodegradable polymers, to form therapeutic compositions.

"Pharmaceutically acceptable" means it is, within the scope of sound medical judgment, suitable for use in contact with the cells of humans and lower animals without undue toxicity, irritation, allergic response and the like, and are commensurate with a reasonable benefit/risk ratio.

In the pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Such unit administration form is itself another object of the invention. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. Generally, the form is sterile and fluid to the extent that easy syringability exists. It is stable under the conditions of manufacture and storage and is generally preserved against the contaminating action of microorganisms, such as bacteria and fungi.

Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations may contain a preservative to prevent the growth of microorganisms.

The TSLP antagonist can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

Preferably said TSLP antagonist, and optionally said immunostimulating agent, is administered in a therapeutically effective amount.

By a "therapeutically effective amount" is meant a sufficient amount of the TSLP antagonist, and optionally of said immunostimulating agent, to provide therapeutic benefits, and particularly to treat and/or to prevent a chronic viral infection, at a reasonable benefit/risk ratio applicable to any medical treatment.

It will be understood that the total daily usage of the antagonists, agents and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidential with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

### Method of prognosing the outcome of cervix dysplasia associated with HPV infection

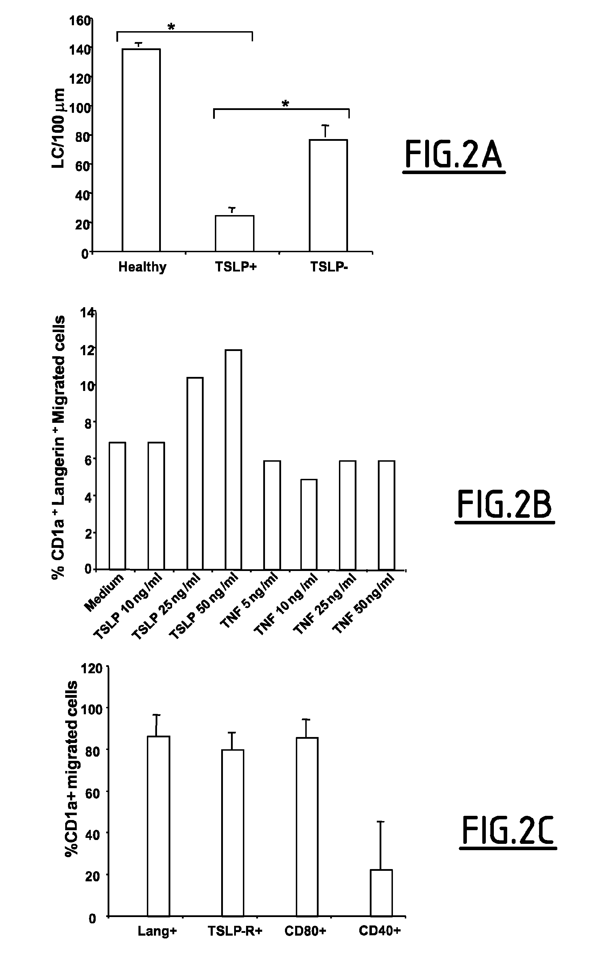

The inventors observed that whereas all examined warts (veruca vulgaris, plantaris and plana) associated with HPV infection exhibited TSLP expression, TSLP expression could not be detected in all cervical dysplasia (see Figure 2A).

Thus, the invention relates to a method of determining if TSLP is expressed in a cervical dysplasia, which method comprises the step of detecting TSLP expression in a sample of said cervical dysplasia. Said method may further comprise the steps of detecting TSLP expression in a control sample, and of comparing the level of TSLP expressed in the sample of cervical dysplasia with the level of TSLP expressed in the control sample.

Since TSLP is implicated in immune evasion during HPV infection, detecting TSLP expression in a cervical dysplasia should be indicative that the cervical dysplasia is likely to persist or to progress towards a cervical intraepithelial neoplasia and cervical cancer.

Accordingly, the invention also provides for a method of prognosing evolution of a cervical dysplasia, which method comprises the step consisting of detecting TSLP expression in a sample of a cervical dysplasia, wherein if TSLP expression is detected then the cervical dysplasia is likely to persist or to progress towards a cervical intraepithelial neoplasia and cervical cancer, and if no TSLP expression is detected then the cervical dysplasia is likely to regress.

The sample of a cervical dysplasia may have been obtained according to any suitable mean, such as biopsy of the cervix, in particular in a subject infected with HPV, more particularly type-16 HPV or type-18 HPV.

A control sample may consist of a sample of basal layer of undifferentiated keratinocytes and of dermis. The control sample may have been obtained from the subject who was submitted to cervix biopsy, to obtain the sample of a cervical dysplasia, or from another subject.

The phrase "detecting TSLP expression" refers to any quantitative, semiquantitative, or qualitative method of detecting TSLP protein or mRMA or of detecting TSLP activity.

As used herein, TSLP is considered to be expressed in a cervical dysplasia if TSLP expression can be detected, and preferably if the level of TSLP is significantly increased by comparison with the level of TSLP measured in a control sample. In such cases, the cervical dysplasia is said to be "TSLP-positive". A significant increase in the level of TSLP expression preferably denotes an increase of at least 10%, preferably at least 20%, more preferably at least 30%, more preferably at least 40%, still preferably at least 50%.

If no TSLP expression can be detected, or preferably if the level of TSLP is not significantly different from the level of TSLP measured in a control sample" the cervical dysplasia is "TSLP-negative".

TSLP expression can readily be detected by the skilled person according to methods conventional in the art, by detecting or measuring TSLP mRNA or protein expression, e.g. by *in situ* immunohistochemistry or immunofluorescence.

For instance, TSLP protein can be detected *ex vivo* with an anti-TSLP antibody, preferably conjugated to a detectable label.

The term "label" refers to an identifying tag that can be attached to a carrier substance or molecule (such as an antibody or oligonucleotide) and used to detect TSLP. A label may be attached to its carrier substance directly or indirectly by means of a linking or bridging moiety. Suitable labels include, but are not limited to, enzymes, e.g., beta-galactosidase, peroxidise or alkaline phosphatase, fluorescent compounds, e.g., rhodamine, fluorescein isothiocyanate, phycoerythrin (PE), Texas Red, Peridinin chlorophyll protein (PerCP) or FITC, luminescent compounds, e.g.; dioxetanes, luciferin, radioactive isotopes, e.g., ¹²⁵I, protein-binding partners, e.g., biotin, and the like.

Methods of detecting TSLP protein according to this embodiment comprise contacting a cervical dysplasia sample with an anti-TSLP antibody, binding the antibody to TSLP, and detecting a complex formed by the antibody and TSLP.

Where the antibody used as probes for identifying TSLP in cells, tissues of cervical dysplasia sampleis labeled with a fluorescent dye, immunofluorescence microscopy may be used to detect the complex formed by the antibody and TSLP. An alternative to immunofluorescence for detecting TSLP protein in tissue sections is immunohistochemistry, in which the specific antibody is chemically coupled to an enzyme that converts a colorless substrate into a colored reaction product which is insoluble and precipitate in situ, i.e. at the site where it is formed.. The localized deposition of the colored product where antibody has bound can be directly observed under a light microscope. Horseradish peroxidase and alkaline phosphatase are the two enzymes most commonly used in these applications. Horseradish peroxidase oxidises the substrate diaminobenzidine to produce a brown precipitate, while alkaline phosphatase can produce red or blue dyes depending on the substrates used; a common substrate is 5-bromo-4-chloro-3-indolyl phosphate plus nitroblue tetrazolium (BCIP/NBT), which gives rise to a dark blue or purple stain.

Immunoblotting (Western blotting) may also be used for identifying the presence of TSLP protein or mRNA in cell lysates. Unlabeled cells are placed in detergent to solubilize all cell proteins and the proteins of the lysate are separated, e.g. by running the lysate on SDS-PAGE, then transferred to a stable support such as a nitrocellulose membrane. TSLP proteins are detected by treatment with antibodies and the bound antibodies may be revealed by anti-immunoglobulin antibodies labeled with radioisotopes or an enzyme.

Similarly, Northern blotting may be employed to detect TSLP mRNA in size-separated RNA using a detectable probe specifically hybridisable, e.g. complementary, to TSLP mRNA.

The invention will be further illustrated in view of the following figures and examples.

### FIGURES

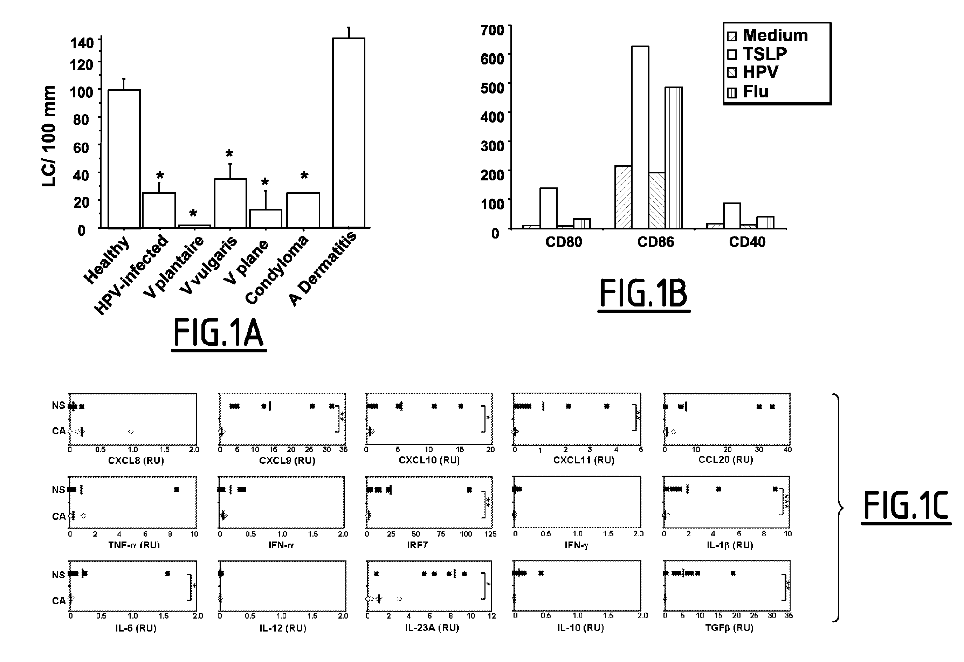

Figure 1 depicts the effects of a HPV infection on the LC from the epithelium. Figure 1A LCs were counted on tissue sections. Data are shown as mean +/- SD for healthy skin (n=6), pooled HPV lesions (n=31), *verruca plantaris* (n=8), *verruca vulgaris* (n=6), *verruca plana* (n=7), *condylomata acuminata* (n=10), and atopic dermatitis (n=5). (* p < 0.05). Figure 1B : level of DC activation based on the surface expression of CD80, CD86 and CD 40 during infection of DC with HPV or Flu, or by contact with TSLP. Figure 1C : effect of a HPV infection on the expression of various cytokines and chemokines (NS: normal skin, CA: condyloma affected skin).
Figure 2 depicts the effect of TSLP on LC cells migration. Figure 2A : LCs were counted in healthy uterine cervical epithelium (n=9), TSLP positive (n=35) and TSLP negative (n=12) CIN1 lesions. Data are shown as mean +/- SD (* p < 0.05). TSLP-positive lesions of the cervix display reduced LC counts as compared with TSLP-negative lesions of the cervix and healthy cervix. Figure 2B : dose dependent effect of TSLP on LC migration. Figure 2C : surface marker expression of CD1a⁺ migrated cells.

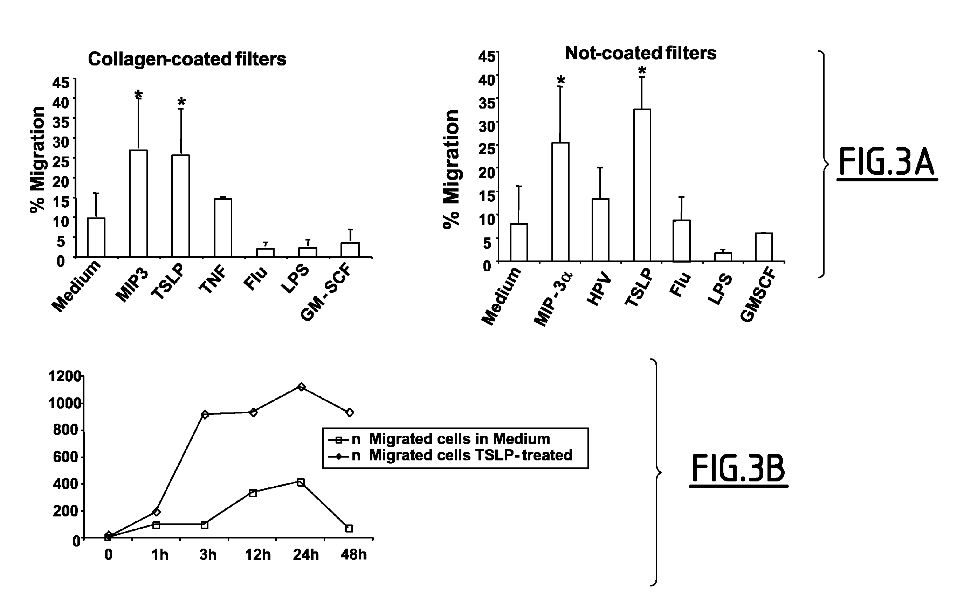

Figure 3 depicts how TSLP acts on DC cells. Figure 3A : migratory capacity of DC after activation with TSLP, TNF, TLR, LPS or influenza virus in uncoated filters or collagen-coated filters to mimic "free movement" or "three dimensional movement". Figure 3B : time needed by the DC to begin their migration after treatment with TSLP.

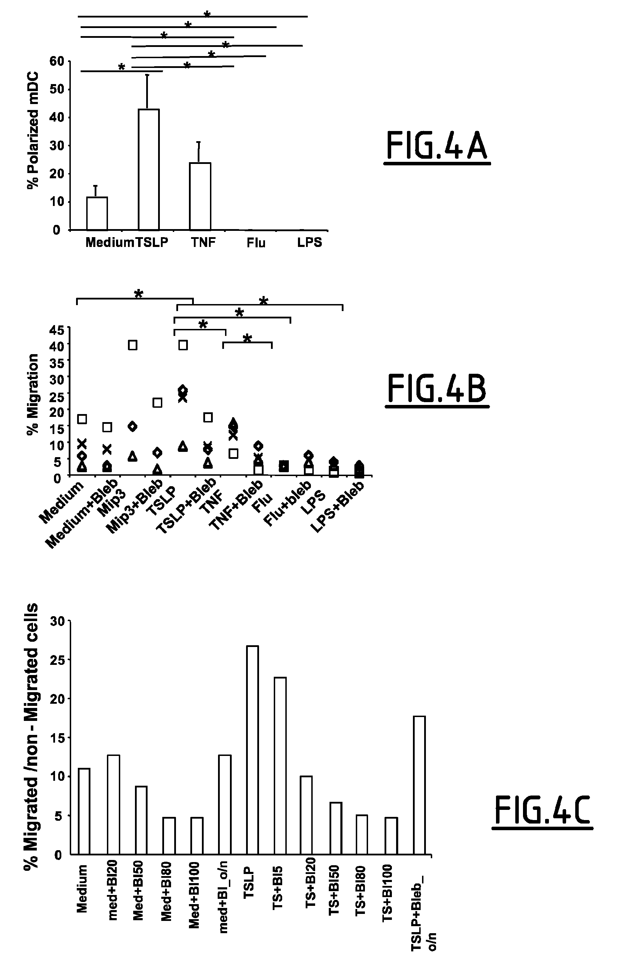

Figure 4 shows that TSLP-induced DC polarization is myosin II-dependent. Figure 4A : polarization of DC, estimated from the positions of the actin cytoskeleton in the cell and the position of the podosomes over the cell surface, after infection or treatment with TSLP, TNF, Flu or LPS. Figure 4B : effect of Blebbistatin, a myosin II inhibitor, on the ability of TSLP, Mip3α (CCL20), TNF, Flu or LPS to induce DC cell polarization. Figure 4C : dose of Blebbistatin which is necessary to inhibit the TSLP induced cell polarization.

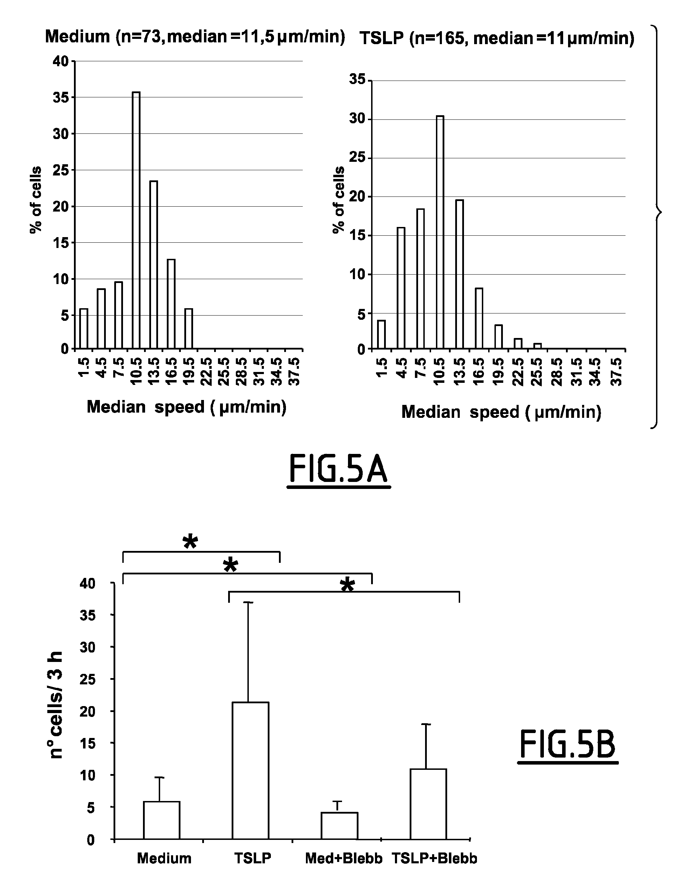

Figure 5 illustrates TSLP effect on DC motility in a confined environment. Figure 5A : in a micro-channel system, TSLP activation has no effect on the velocity of the DC comparing to non TSLP-activated DC. Figure 5B: the number of DC entering the channels during a 3 h time-lapse movie was quantified when cells were pre-cultured in the absence or presence of blebbistatin (50 nM). TSLP induced a 4 fold increase in the capacity of DCs to enter micro-channels, as compared to control medium. Blebbistatin significantly inhibited this effect. Data are shown as mean +/- SD, n=3 (* P < 0.05).

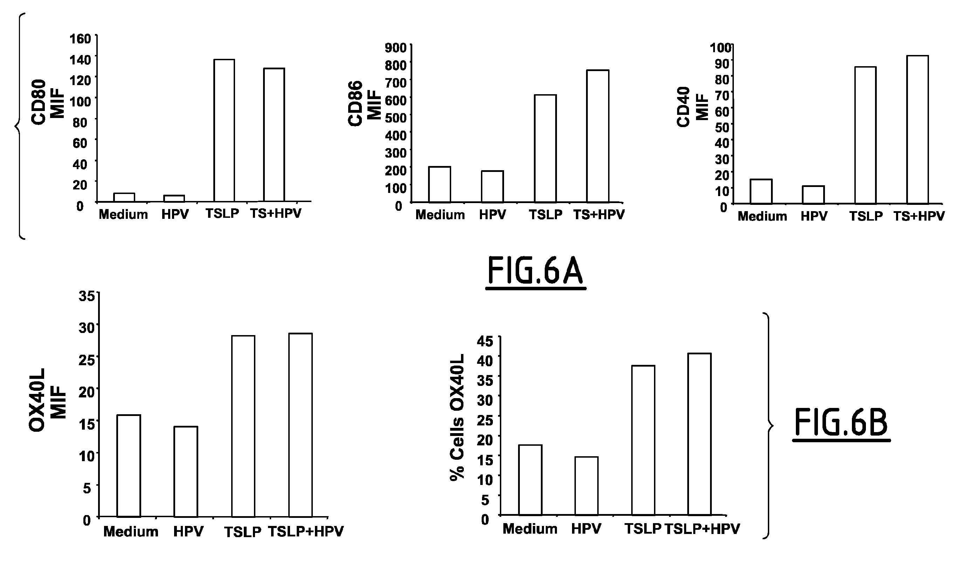

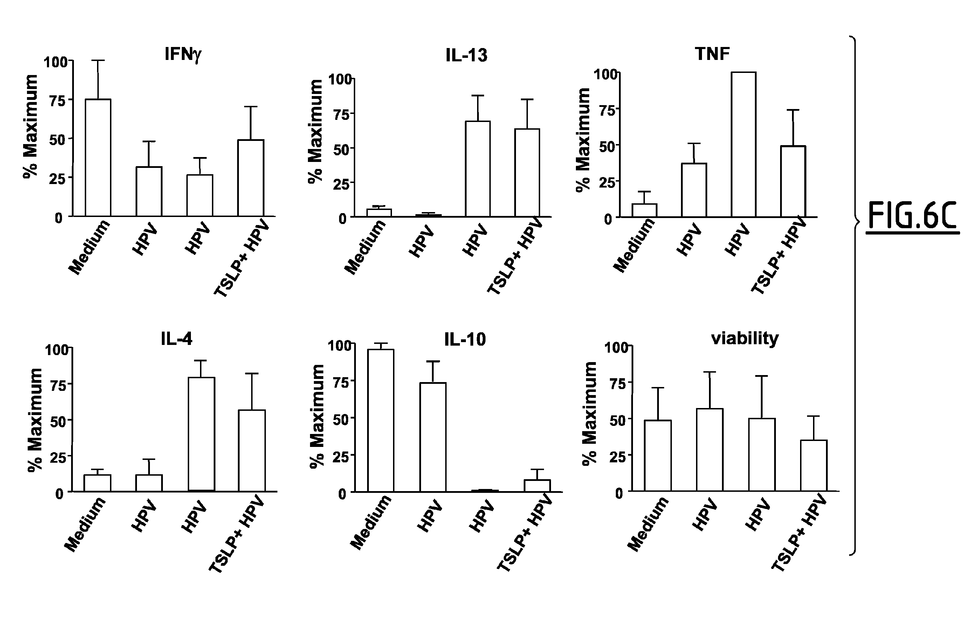

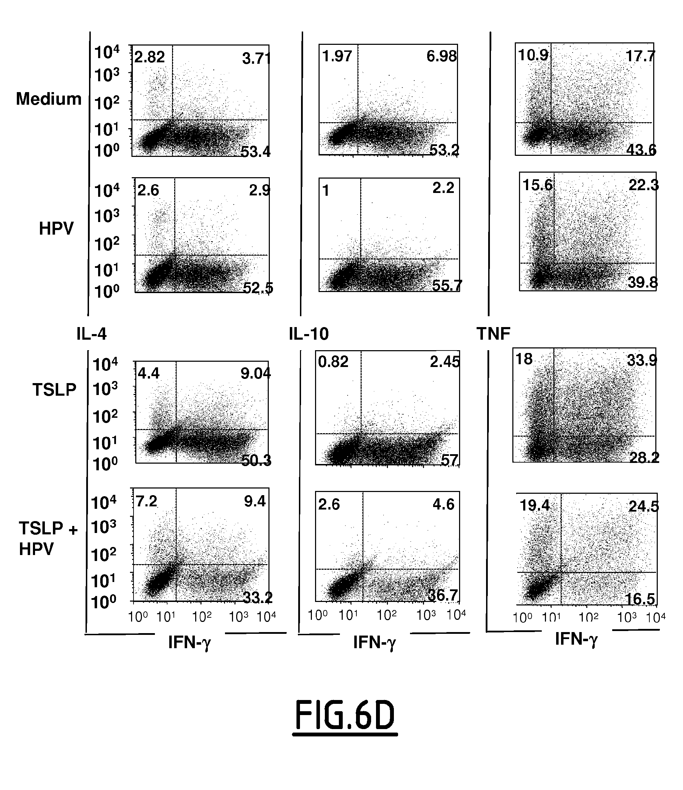

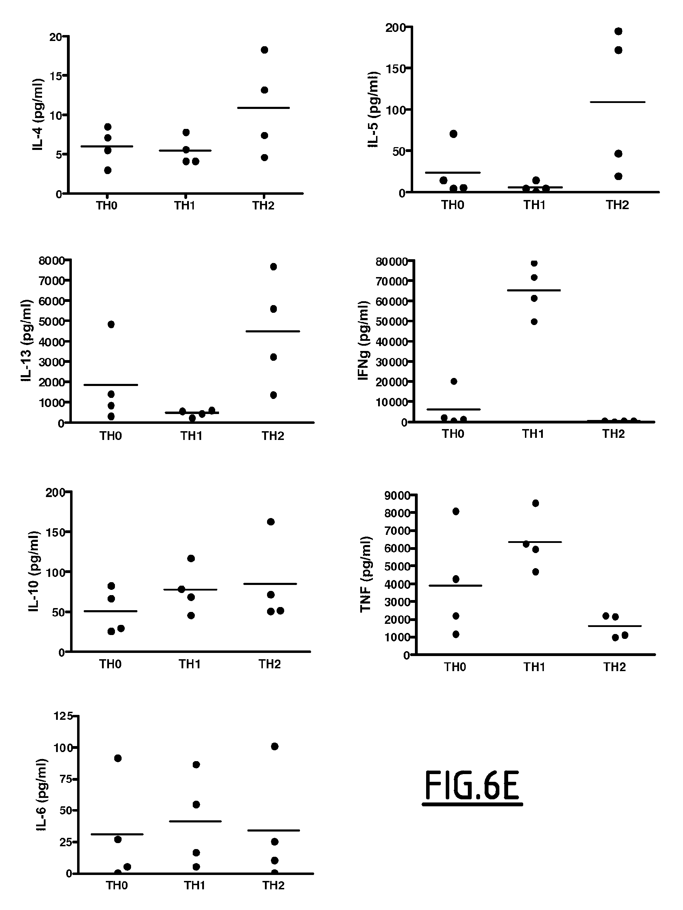

Figure 6 shows that TSLP drives a TH2 response even in presence of HSBV. Figure 6A : after 24 h of culture, HPV did not induce any DC activation based on surface levels of CD40, CD80 and CD86. TSLP induced a strong up-regulation of these 3 maturation markers, which was not affected by HPV. MFI: Mean Fluorescence Intensity. Figure 6B: after 48 h of culture, TSLP induced an up-regulation of surface OX40-ligand (OX40L) expression on DC (left panel) and of the proportion of DCs expressing OX40L (right panel). MFI: Mean Fluorescence Intensity. Figure 6C : production of INF-γ, IL13, IL4, IL10, TNF, by T cells induced by DC activated by HPV, TSLP or HPV+TSLP. Figure 6D : production of IL4, IL10, TNF, INF-γ by T cells induced by DC activated by HPV, TSLP or HPV+TSLP by FACS. Figure 6E : Naive T helper cells were cultured for 5 days with anti-CD3+anti-CD28 in the absence or presence of polarizing cytokines and the Th cytokines TNF, IL-4, IFN-γ IL-13, and IL10 were measured after a subsequent 24 h polyclonal re-stimulation. TH0: no polarizing cytokine added; Th1: IL-12; Th2: IL-4. Each dot represents values from independent experiments. Bars represent the mean.

### EXAMPLES

### Example 1: Depletion of LCs is an inherent attribute of HPV infection

In order to determine if LC depletion was a general feature in all types of HPV infection, the number of LCs in different types of warts (veruca vulgaris, plantaris and plana) as well as condyloma was quantified in comparison with normal skin.

The pool of LC almost disappeared in every cutaneous lesion studied (Fig 1A). Therefore, depletion of LCs is an inherent attribute of HPV infection. It was, thus, hypothesized that a factor present in the HPV microenvironment would induce the activation and migration of LCs.

### Example 2: HPV cannot directly infect and/or activate DC

The capability of HPV to directly infect and/or activate DC was analyzed. Because of cell number limitations, primary DCs directly isolated from the blood of healthy donors were used. They share many similarities with LC. Advantage was taken of the possibility to use whole virus HPV-1 as well as virus-like particles (VLP) 16 and VLP 18.

Whole primary HPV-1 virions were purified from plantar warts as described in Orth et al. (J Virol, 1977, 24, 108-120). VLP16 and VLP 18 were a kind gift of Glaxo-Smith Kline. To assess HPV entry into DCs, cells were incubated with 10⁷ HPV-1 virus particles/ml or 10 µg/ml VLP 16 or VLP 18 for 24 h. DCs were then washed and cytospined at 7000 x for 10 min. Slices were then frozen on dry ice, fixed in cold acetone (-20°C) for 10 min and stored at -80°C until use for virus detection by immunofluorescence. HPV and virus particles were detected by using and mouse anti-L1 protein antibody (Visoactiv & Virofem) followed by an antimouse IgG couple to the Cy3 flourochrome (Jackson ImmunoResearch Lab).

After 24 h of incubation, HPV-1 was able to enter DCs but did not induce DC activation based on surface expression of CD40, CD80 and CD86 (Fig 1 B). Similar data were obtained with *ex vivo* or *in vitro* generated LC or using VLP. Thus, a direct activation of DCs by HPV could be excluded.

### Example 3: Cytokines expression during HBV infection

Next, it was hypothesized that LCs activation and migration could be induced by proinflammatory cytokines present in the microenvironment of HPV infected tissues (Cumberbatch et al., Clin. Exp. Dermatol. 2000, 25(5):413-8, Cumberbatch et al., Br. J. Dermatol., 1999, 141 (2):192-200). Condyloma was used, as a model HPV lesion, and the gene expression of pro-inflammatory cytokines and chemokines were analyzed.

As compared to normal skin, comparable levels of TNF-α, IFN-g and IL-12 were observed, whereas IL-1b, IL-6 and IL-23 were significantly decreased (Figure 1 C). Thus, no cytokine candidate could be identified for DC activation in these profiles.

An absence or decreased levels of various chemokines was also found (Fig 1C), in particular in the CCL20 production, which was confirmed by inmunohistochemistry. CCL20 downregulation was also described in cervical lesions (47) and might play a role in the impaired recruitment of LC precursors (45). Interestingly, the anti-inflammatory cytokines IL-10 and TGF-b were also down-regulated as compared to normal skin, suggesting that they were not implicated in immune evasion to HPV and contrasting with previous reports.

Strikingly, high levels of TSLP were found in HPV infected lesions of the skin and cervix. As previously observed in atopic dermatitis, TSLP production was absent in the basal layer of undifferentiated keratinocytes and there was no TSLP staining in the dermis.

### Example 4: TSLP implication in DC migration

To address the role of TSLP in LC migration in the context of HPV infection, advantage was taken of the fact that approximately 30% of CIN-1 lesions did not express TSLP at the time of biopsy. The TSLP expression was correlated with the number of epidermal LC, and it was found that only infected cervix devoid of TSLP was able to maintain the pool of LCs (Fig 2A). This suggested that TSLP may trigger LC migration.

Next, epidermal explants were used as a global model to study the emigration of LC. TSLP significantly increased the migration of CD1a⁺ Langerin⁺ TSLP-receptor⁺ CD80⁺ cells (FIG 2B and C). In this respect, TSLP was more efficient than TNFa, a cytokine considered as very potent in inducing LC migration (Cumberbatch et al., Clin. Exp. Dermatol., 2000, 25(5):413-8; Cumberbatch et al., Br. J. Dermatol. 1999, 141 (2):192-200).

In the skin explant model, TSLP effect could be indirect or favoured by factors produced by keratinocytes. *In vitro* transwell experiments were performed to analyse in details the ability of TSLP-activated DC (TSLP-DC) to migrate. It was attempted to mimic two types of movement: (i) a "free-movement" when cells were let to migrate through uncoated filters and (ii) a "three-dimensional movement" closer to the *in vivo* situation using collagen I-coated filters.

To that end, uncoated or collagen Type I (5 µg/ml rat tail collagen type I, BD Biosciences) coated transwells (Costar, 3 µm pores) were placed in 96-well plates filled with 200 µl of DC culture medium.

CD11c⁺ DCs were purified to 99% by FacSorting from buffy coats of healthy adult volunteer blood donors (Crozatier blood bank, Paris, France) as previously described (Soumelis et al., Nat Immunol, 2002, 3, 673-680). Freshly sorted CD11c⁺ DCs were cultured in RPMI containing 10% fetal calf serum, 1% pyruvate, 1% HEPES and 1% penicilin-streptomycin. Cells were seeded at 1 x 10⁶/ml in flat-bottom 96-well plates in the absence (untreated cells) or presence of 50 ng/ml TSLP (R&D Systems), 10⁷ HPV particles, 2.5 ng/ml TNF (R&D), 20 µg/ml influenza virus (H1N1, A/PR/8/34 strain, Charles River Lab.), 1 µg/ml LPS (Sigma), or 100 ng/ml GM-CSF (BruCells).

Overnight treated DCs (1 x 10⁶/ml) with TSLP, TNF, LPS, influenza virus or GM-CSF were re-suspended and 50 µl of this solution and were added to the upper chamber of the transwells and incubated at 37°C for 6h. MIP-3α/CCL20 (500 ng/ml) (R&D) was added to the lower chamber as a positive control to induce DC migration where mentioned. After 6 h, cells in the upper and the lower chamber of the transwell were counted. In some experiments, DCs were pretreated with 200 ng/ml pertussis toxin during 24 h and/or the 6 h of migration time. Results were expressed as % of total DCs.

TSLP-DCs became highly efficient for migration in both systems (Fig 3A). Migratory capacity of TSLP-DC was higher that TNF-DCs. Two Toll-like receptor (TLR) ligands, LPS and influenza virus, were unable to induce DC migration (Fig 3A). TSLP-induced migration started as soon as 3 hours after TSLP exposure, in accordance with the expression of TSLP receptor by human DC in culture (FIG 3B). Thus, TSLP can potently induce migration by acting directly on the DC, independently of chemokines.

### Example 5: TSLP induced a myosin II dependent polarisation of human DC with an important re-organization of the cell cytoskeleton

To address the underlying molecular mechanisms, cell polarization was analyzed, as a characteristic of cell activity and migration that has to be regulated for acquiring movement. Actin cytoskeleton is the machinery required for cell expansions and actin reorganization and is essential for cell polarization and movement.

To determine the cytoskeleton architecture, DCs were cultured on poly-lysine-coated coverslips for 24 h and examined by epifluorescence microscopy. Cells were fixed in 4% PFA in phosphate-buffered saline (PBS) for 20 min at room temperature, permeabilized by 1% Triton X-100 in PBS for 5 min, and blocked with 1% bovine serum albumin (BSA) in PBS for 20 min at room temperature. For localization of filamentous actin, cells were incubated with Cy3-phalloidin (Molecular Probes) for 30 min. Counting of number of polarized DCs from 5 different donors assessed polarization index. Polarization was expresses as proportion polarized cells respect to total number of cells. Localization of α-tubulin was achieved by incubation for 1 h with a rat anti-human α-tubulin antibody (Serotec). Myosin II was detected by a rabbit anti-human myosin II heavy chain antibody (BTI) followed by incubation for 30 minutes with Alexa 488 goat anti-rabbit (Molecular Probes). Coverslips were mounted in ProLong Gold antifade reagent (Invitrogen). Fluorescence images were obtained by means of an epifluorescence microscope (Leica) fitted with appropriate filter sets.

Non stimulated human DC in poly-lysine-coated coverslips appeared non-polarized, with actin cytoskeleton organized in the periphery of the cell and enriched in podosomes diffusely distributed over the cell surface. When activated by influenza virus, the same non-polarized morphology was maintained. LPS induced the formation of multiple dendritic expansions together with a loss of podosome and the cell acquired a "stellar" shape. Interestingly, cells became extremely polarized after TSLP treatment (Fig 4A) with a well-developed leading edge where the nucleus was displaced and a long very thin uropod at the other cellular pole. Podosomes were clustered predominantly in the leading area and/or actin-filaments reinforced around the nucleus. TNF-mDC also adopted a polarized shape, although polarization was less obvious (Fig 4A). Polarized growth of microtubules is also crucial for cell polarization. Similarly to actin cytoskeleton, microtubules were organized in a non-polarized manner in human DC cultured with medium and this shape was unchanged in the presence of influenza virus. LPS-treatment induced a reorganization of the microtubules in dendritic expansions. Cellular shape became polarized also respecting microtubule skeleton in TSLP and TNF-α-stimulated DC. In conclusion, TSLP induced the polarization of human DC with an important re-organization of the cell cytoskeleton.

The member of the non-muscle myosin family myosin II is a motor protein capable of binding actin and is directly implicated in cellular expansion and cell movement. Myosin II consists in 2 heavy chains in which the N-terminus forms a globular head with actin- and ATP-binding sites, and 2 light chains. After actin binding, myosin II is able to move to the plus-end of actin filaments and induces actin filament contraction. In TSLP-DC, but not medium or LPS-DC, myosin II accumulated at the leading edge together with actin filaments suggesting cell retraction. In conclusion, TSLP-treatment drove mDC maturation, polarization and actin-myosin II re-localization together with migratory abilities.

Given the intense reorganization of the actimyosin cytoskeleton, the question was asked whether TSLP-induced DC migration was myosin II-dependent. Blebbistatin is a small molecule inhibitor that blocks the head of the myosin II in an actin-detached state. To study the role of myosin II in the morphology of TSLP-DCs, cells were incubated for 12 h in TSLP with or without 20 nM blebbistatin on poly-lysine-coated slides to permit the polarization of cells. Low concentration was chosen to avoid the toxic effect of blebbistatin.

Inactivation of myosin II inhibited the polarization and migration induced by TSLP (Fig 4B). DC migration was inhibited by dose as low as 20 nM blebbistatin (Fig 4C). The reduction in migration was accompanied by a loss of the well-organized polarized morphology. Blebbistatin treatment resulted in an extremely elongated cellular shape previously described as non-physiological. Thus, TSLP-induced DC migration and polarization of the cell cytoskeleton are myosin II-dependent.

### Example 6: TSLP promotes DC motility in a confined environment

A tissue represents a confined environment for cell migration (Irimia et al., Lab Chip, 2007, 7, 1783-1790). Cells may be "trapped" in narrow spaces, being forced to pass through areas of diverse densities. In order to mimic such *in vivo* situation, a micro-channel system was used. This system allows quantifying diverse parameters to define the cell movement and to restrain the direction of the mobile cells.

The microfluidic device was fabricated in PDMS (Whitesides G. M., E., O., Takayama S., X., J. & E, I.D. Ann Rev Biomed Eng, 2001 3, 335). The PDMS piece, with embedded microchannels and holes for the inlet and outlet ports, and a glass Iwaki chamber (Milian) were activated in a plasma cleaner (PDC-32G Harrick) and bonded to each other. The chambers were left under strong vacuum for 5 min in the plasma cleaner and plasma was turned on to render the top surface of the PDMS and the inlet and outlet holes hydrophilic. Fibronectin solution at 50 µg/ml was placed on top of the inlet and outlet ports. The solution spontaneously invaded the channels and all air bubbles were resorbed into the PDMS due to the previous vacuum treatment. Fibronectin was incubated for 1 h at room temperature, then washed with PBS then replaced by cell culture medium. The cells were concentrated and micropipette tips containing the cells were inserted in the inlet port. Cells fell inside the port, bound to the bottom coverslip and started migrating. They entered the channels spontaneously, without any mechanical or chemical stimulation.

Phase contrast images at various positions in the chambers were recorded with time-lapses of to 2 min during 6 h, using an automated microscope (Nikon ECLIPSE TE1000-E, and Olympus X71, with a Marzhauser motorized stage and an HQ2 Roper camera) equipped with an environmental chamber for temperature, humidity and CO₂ (Life Imaging Services). Cells remained alive and motile during the entire period of recording.

To analyse the importance of myosin II in DCs migration, cells were pretreated with 50 nM blebbistatin and then concentrated and inserted in the microchannels.

First, the median velocity of DC was measured and no significant difference was found between DC pre-cultured in medium or TSLP (Fig 5A). Similar results were obtained for maximal and minimal DC velocities This indicated that the increased migration observed with TSLP in the transwell system was not due to increased speed. By observing live imaging of DC migration, it was noticed that TSLP-DCs were more competent in reaching the border and entering the micro-channels. As a result, more DC were travelling inside the channels at a given time after TSLP pre-treatment as compared to medium. The TSLP-induced increase in DC entering inside the channels was myosin-11 dependent (Fig 5B) and was not observed in other DC activating conditions.

This indicates that TSLP promotes DC motility in a confined environment and suggests that it favors the initiation of the movement and the passage through narrow gaps, in accordance with a recent report demonstrating a role for myosin II in the three-dimensional movement of mouse leukocytes (Lämmermann et al., Nature 2008; 453(7191):51-5). This contrasts with the invariant chain control of DC motility, which affects the velocity and type of DC movement but not the entry into similar microchanels.

### Example 7: TSLP triggering may drive a Th2 response

The results herein described provide a molecular basis for the LC depletion observed in HPV infection and contributing to the local immune suppression. However, an important question is the fate of migrating TSLP-activated DC and their ability to induce an antiviral T cell response. TSLP is known to induce a pro-allergic Th2 response. It was asked, whether HPV was able to modulate TSLP-induced DC activation and subsequent T cell priming.

TSLP induced a potent activation of DC, based on surface expression of co-stimulatory molecules, which was not modified in the presence of HPV (Fig 6A and 6B).

CD11c⁺ DCs were purified to 99% by FacSorting from buffy coats of healthy adult volunteer blood donors (Crozatier blood bank, Paris, France) as previously described (Soumelis et al., Nat Immunol, 2002, 3, 673-680). Freshly sorted CD11c⁺ DCs were cultured in RPMI containing 10% fetal calf serum, 1% pyruvate, 1% HEPES and 1% penicilin-streptomycin. Cells were seeded at 1 x 10⁶/ml in flat-bottom 96-well plates in the absence (untreated cells) or presence of 50 ng/ml TSLP (R&D Systems), 10⁷ HPV particles, TSLP+HPV.

After 24 h of culture, stimulated CD11c⁺ DCs were collected, washed, and co-cultured with allogenic naïve CD4⁺ T cells in round-bottomed plates 96-well culture plates (Falcon) at a 1:5 DC: T cell ratio in Yssel's medium (kind gift of Hans Yssel) containing 10% FCS (Hyclone). Peripheral blood naïve CD4⁺ T cells were isolated by using CD4 T cell isolation kit II (Miltenyi Biotec) followed by staining for CD45RO-FITC, CD45RA-PE, CD4-APC, and cell sorting of CD45RA⁺, CD4⁺, CD45RO⁻ cells (purity > 99%) with a FACSAria (BD Bioscience). Standard Th subsets were generated in presence of Dynabeads CD3/CD28 T cell expander (1 bead per cell) (Invitrogen) and 10 ng/ml IL-12 (R&D Systems) for Th1, 25 ng/ml IL-4 (R&D Systems) for Th2 and in absence of any polarizing cytokine for Th0.

After 5-6 days, cells were harvested, extensively washed and viability was determined by trypan blue exclusion. 1x10⁶ cells/ml were re-stimulated with Dynabeads CD3-CD28 T cell expander (1 bead per cells) for 24 h (ELISA) or with 100 ng/ml PMA and 1mg/ml lonomycin for 6 h (FACS intracellular staining). Cytokines in culture supernatants were measured by cytometric bead assay (CBA) Flex Sets (BD Bioscience) according to the manufacturer's instruction. IFN- γ-, IL-4-, IL-10-, TNF-producing cells were analysed by intracellular cytokine staining after addition of 10 µg/ml Brefeldin during the last 3 h of re-stimulation. Cells were permeabilized using Cytofix-Cytoperm reagents (BD Biosciences). Cells were stained with anti-IFN-γ FITC, anti-IL-4 PE, anti-IL-10 PE, anti-TNF PE (BD Pharmingen) washed and then analysed by flow cytometry (FACScan Becton Dickinson).

When TSLP-DCs were used to stimulate naive CD4+ T cells *in vitro,* a Th2 profile was observed, with production of IL-4, IL-5, and IL-13 together with TNF-α, whereas the T cell cytokine profile induced by HPV-DC was similar to medium (Fig 6C and D). When TSLP and HPV were combined to activate DC, the subsequent T cell cytokine profile was similar to TSLP-DC, indicating that TSLP was dominant over HPV. This suggested that residual LC or DC migrating to the draining lymph node following TSLP triggering may drive a Th2 response that is not appropriate for viral clearance.

In conclusion, the results provided herein for the first time implicate TSLP in the physiopathology of a viral infection. The local microenvironment is proposed to have critically affects the outcome of TSLP-driven DC activation, from pro-inflammatory in allergy to promoting immune evasion in HPV infection.

Importantly, HPV did not prevent TSLP from priming for a Th2 response, which is not appropriate for viral clearance (Kawai and Akira, Nature Immunol, 2006, 7, 131-137). This contrasts with the direct TLR-dependent DC activation induced by other viruses (Kawai and Akira, Nature Immunol, 2006, 7, 131-137), such as influenza virus or HSV, which leads to a protective Th1 response and the eradication of the infection (Alcami, Nat Rev Immunol, 2003, 3, 36-50).

Langerhans cells (LCs), the resident epidermal DCs, play an important role in the defence against viral infections, such as HIV and herpes simplex virus (HSV). It was previously shown that LCs were depleted from HPV-infected uterine cervical epithelium, which may create a state of local immune suppression and has been considered as a strategy of immune evasion (Stanley, Vaccine, 2006, 24, S16-S22). However, the factors triggering the emigration of LCs have remained unknown. It is demonstrated that LC depletion is a general feature of skin and mucosal HPV infection and provide evidence for a critical role of TSLP in this process.

The data described herein indicate that induction of DC migration is an intrinsic property of TSLP independently of the inflammatory context. Interestingly, LCs are depleted only in HPV infection and not in Atopic Dermatitis, despite the presence of TSLP in both types of lesions. This can be attributed to the differential expression of chemokines, such as CCL20, which are important for the recruitment of new LCs or LC precursors to the epithelium. In cervical dysplasia, there is a lack of CCL20 (MIP-3 α) (Guess and McCance, J Virol, 2005, 79, 14852-14862), whereas this chemokine is upregulated in AD (Dieu-Nosjean et al. J Exp Med, 2000, 192, 705-718). Thus, the impact of TSLP on the global outcome of the immune response is partly dependent on the microenvironment.

Cell motility is a fundamental characteristic of DCs that enable them to emigrate from peripheral tissue after antigenic challenge and to reach the secondary lymphoid organs ⁶. TSLP was identified as a novel factor able to directly trigger DC migration in a confined environment during the course of HPV infection. A micro-channel system that mimics the microenvironment encountered by DCs in the constrained interstitial spaces of skin was used. In addition, the cellular characterization of TSLP-induced human DC migration revealed a novel molecular mechanism of cytokine-induced migration that depends on myosin II. These results provide a strong link between fundamental mechanisms of DC migration in a confined environment with the physiopathology of a human viral infection.

Importantly, these results provide an explanation for the initiation of HPV-specific immune responses, thus reconciling a long-lasting paradox. Indeed, the present model enables to explain how most HPV infections are self-limited and spontaneously regress, even after months or years, and how HPV-specific immune responses can be initiated. Initially, TSLP may activate resident LCs and enable the initiation of anti-HPV immunity whereas on a longer term, TSLP may subvert the immune response by promoting chronic LC depletion and immune deviation towards a Th2 response, which is not appropriate for efficient viral clearance. The balance between immunity and immune subversion mechanisms would ultimately determine the outcome of the lesion, as is observed for some common warts, or persistence for many months or years, as is the case for uterine cervical lesions.

Accordingly, it is expected that targeting TSLP function in HPV-infected patients will help redirecting the immune response towards a protective Th1 response.

## Claims

1. A TSLP antagonist for treating or preventing a chronic viral infection.

2. The TSLP antagonist according to claim 1, wherein said chronic viral infection is selected from the group consisting of an infection with human papilloma virus (HPV), hepatitis viruses (HBV, HCV), human immunodeficiency viruses (HIV), and molluscum contagiosum virus (MCV).

3. The TSLP antagonist according to claim 1 or 2, wherein said chronic viral infection is an infection with a high-risk subtype of HPV, preferably type-16 HPV or type-18 HPV.

4. The TSLP antagonist according to any one of claims 1 to 3, wherein said TSLP antagonist selectively binds to either TSLP or to TSLPR, the complex receptor TSLPR/ IL-7R alpha chain, or the TSLPR or IL-7R alpha subunit of the complex receptor TSLPR/ IL-7R alpha chain.

5. The TSLP antagonist according to claim 4, wherein the TSLP antagonist is selected from the group consisting of antibodies or aptamers which bind to TSLP, antibodies or aptamers which bind to TSLPR, to the complex receptor TSLPR/ IL-7R alpha chain, or to the TSLPR or IL-7R alpha subunit of the complex receptor TSLPR/ IL-7R alpha chain, soluble TSLP receptor, soluble IL-7R alpha chain.

6. The TSLP antagonist according to any one of claims 1 to 3, wherein the TSLP antagonist reduces or prevents expression of TSLP, TSLPR or the complex receptor TSLPR/ IL-7R alpha chain.

7. The TSLP antagonist according to claim 6, wherein the TSLP antagonist comprises an antisense oligonucleotide interfering messenger RNA or ribozyme.

8. A combination of at least one TSLP antagonist and at least one immunostimulating agent, or a composition thereof, as a medicament, wherein the TSLP antagonist and immunostimulating agent are intended to be administered simultaneously or sequentially.

9. The combination of claim 8, for treating or preventing a chronic viral infection.

10. The combination according to claim 9, wherein said chronic viral infection is selected from the group consisting of and infection with human papilloma virus (HPV), preferably a high-risk subtype of HPV, hepatitis viruses (HBV, HCV), human immunodeficiency viruses (HIV), and molluscum contagiosum virus (MCV).

11. The combination according to claim 9 or 10, wherein said immunostimulating agent is a Th-1 cytokine or an inducer of the production of a Th-1 cytokine.

12. The combination according to any one of claims 9 to 11, wherein said immunostimulating agent is selected from the group consisting of interferon (IFN), inducers of IFN, tumor necrosis factor (TNF), inducers of TNF, interleukin-2 (IL-2), and ligands of Toll-like receptors (TLR).

13. A method of determining if TSLP is expressed in a cervical dysplasia, which method comprises the step of detecting TSLP expression in a sample of said cervical dysplasia.

14. The method according to claim 13, which further comprises the step of detecting TSLP expression in a control sample, and of comparing the level of TSLP expressed in the sample of cervical dysplasia with the level of TSLP expressed in the control sample.

15. A method of prognosing evolution of a cervical dysplasia, which method comprises the step consisting of detecting TSLP expression in a sample of a cervical dysplasia, wherein if TSLP expression is detected then the cervical dysplasia is likely to persist or to progress towards a cervical intraepithelial neoplasia and cervical cancer, and if no TSLP expression is detected then the cervical dysplasia is likely to regress.
